(19) **Europäisches Patentamt / European Patent Office / Office européen des brevets**

(11) **EP 1 762 628 B1**

(12) **EUROPEAN PATENT SPECIFICATION**

(45) Date of publication and mention
of the grant of the patent:
**12.03.2008 Bulletin 2008/11**

(51) Int Cl.:
***C12Q 1/68*** (2006.01)

(21) Application number: **05447202.2**

(22) Date of filing: **13.09.2005**

(54) **Detection method of homologous sequences differing by one base on a microarray**

Verfahren zum Nachweis von homologen Sequenzen, welche sich durch eine Base unterscheiden, auf einem Mikroarray

Procédé pour la détection sur un micro-reseau de sequences homologues différent par une base

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**14.03.2007 Bulletin 2007/11**

(73) Proprietor: **Eppendorf Array Technologies SA 5000 Namur (BE)**

(72) Inventors:
• **Remacle, José**
 **5020 Malonne (BE)**
• **De Longueville, Françoise**
 **5360 Natoye (BE)**
• **Pastoret, Soumya**
 **4020 Liège (BE)**
• **Bertholet, Vincent**
 **5100 Jambes (BE)**
• **Zammatteo, Nathalie**
 **5025 Gelbressée (BE)**

(74) Representative: **Van Malderen, Joëlle et al pronovem - Office Van Malderen Avenue Josse Goffin 158 1082 Bruxelles (BE)**

(56) References cited:
 **WO-A-98/30722        US-A1- 2005 089 877**

• **SEO JINWOOK ET AL: "Interactively optimizing signal-to-noise ratios in expression profiling: project-specific algorithm selection and detection p-value weighting in Affymetrix microarrays" BIOINFORMATICS (OXFORD), vol. 20, no. 16, 1 November 2004 (2004-11-01), pages 2534-2544, XP002368170 ISSN: 1367-4803**
• **KARAMAN MAZEN W ET AL: "Comparisons of substitution, insertion and deletion probes for resequencing and mutational analysis using oligonucleotide microarrays." NUCLEIC ACIDS RESEARCH. 2005, vol. 33, no. 3, 18 February 2005 (2005-02-18), page e33, XP002368171 ISSN: 1362-4962**

**Description**

**Field of the invention**

The present invention is related to a method and kit comprising reagents and means for the identification (detection) of possible mutations (SNPs) in gene(s) or organism genome based on amplification of homologous sequence followed by detection on array.

The invention is especially suited for the simultaneous identification and/or quantification of multiple mutations in the same gene nucleotide sequence or same organism genome.

The present invention is well adapted for diagnostic and analytical assay.

**Background of the invention and state of the art**

The early methods to type single nucleotide polymorphisms SNPs include the following techniques SSCP, RFLP, AS-PCR, sequencing making genotyping judgement coupled with gel electrophoresis. These methods are unfit for large scale screening due to the limitation of detection methods. However multiple analyses of mutations have been facilitated by using DNA microarray based method for questioning each particular position where mutations may occur in a particular sequence. Also the microarray based mutation detection can be extended to multiple different sequences, like typical exons of the same gene which can not usually be sequenced at once given the distance between the exons.

In the most common method, the gene or genome questioned for possible mutation is first amplified and then copied into ribonucleotide sequences in order to be cut into pieces which are then hybridized on oligonucleotides sequences present on the array. The presence of a mutation is considered according to the ratio of the signal of the oligonucleotide having the mutation compared to the wild type.

Several publications exist on this technology. An allele specific oligonucleotide (ASO) based microarray was made for the screening of 4 mutant alleles of CYP2C9 (Wen SY et al. 2003, World J. Gastroenterol., 9, 1342-1346). Pairs of probes with one base difference for SNP discrimination were immobilized on glass slides. Genotype was determined by calculation of the signal ratio of match to mismatch probes. The signal intensity ratio value above 4 or below 2.5 is considered a critical limit for genotyping judgment. When ratio values were between 2.5 and 4, samples were regenotyped.

The patent US6410229 provides an array of nucleic acid probes for SNP detection in RNA transcripts. Quantification of the hybridization is obtained by comparing binding of matched and control probes. The patent application WO9729212 provides a method for identifying a genotype of an organism using an array comprising capture probes complementary to reference DNA or RNA sequences from another organism (for example, using oligonucleotide sequences based on the Mycobacterium tuberculosis rpoB gene). Genotyping is based on an overall hybridization pattern of the target to the array.

The patent US5858659 provides an array comprising detection blocks of probes, each block including four groups of capture probes to question a single base (e.g. use of blocks of 40 oligonucleotides of 20 bases to question one polymorphic base). First and second groups of capture probes are complementary to the target nucleic acid sequence having first and second variants of the polymorphic bases. Third and fourth groups of probes, have a sequence identical to first and second groups of probes, except that they include mono-substitutions of positions in said sequence that are within n bases of the polymorphic base.

If the method is working, it has some drawbacks since different capture probes have different affinity for target sequences and the ratios between the mutated to non mutated capture probes varies a lot from one mutation to the other. Also the determination of common hybridization conditions is a problem with some capture probes having better discrimination than the others. The consequence is a very heterogeneous pattern of ratios and sometimes a difficulty to determine whether the organism is homozygote or heterozygote for the mutations at specific loci.

Two improvements have been recently proposed to this method. The US patent application 2005/0089877, provides a method for genotyping DNA sequence on chips with a wild-perfect match and a mutant perfect match probe. The method proposes a genotyping algorithm for the optimization of the capture probes and a statistical robust method. The algorithm is based on the analysis of data coming from a hybridization of an identified standard nucleic acid and from a genotyping of the unknown target by substituting input vectors into the genotyping algorithm.

The US patent 6,852,488 is related to a sequencing method of a target sequence, but with particular detection of mutation in the target sequence. The method is based upon the use of a core known sequence with high affinity for the target sequence. The method proposes a selection of a probe by evaluating the binding characteristics of all the probes having a single mismatch as compared to a core probe. If the single base mismatch probes exhibit characteristic binding or affinity pattern, then the core probe is exactly complementary to at least a portion of the target sequence. This method allows mutation detection in a target sequence by a comparison of binding affinity of a known core probe for the target sequence with the binding affinity of the probe having a single nucleotide variation. This selection method of the probe is based on the experimental screening of capture probes with selection of one with high affinity binding.

However, these two methods require multiple experimental steps and are therefore complicated and time consuming. Furthermore, they are not fit for the development of large number of mutation detections on microarray since they require experimental optimization for each mutation and they use a different calculation process for obtaining a final result related to each mutation. They also do not provide an a priori solution for developing biochips for the detection of multiple SNPs in one organism genome.

## Summary of the invention

The present invention provides an original and easy solution to determine the presence or absence of at least 3 and preferably 5 and still preferably 20 single nucleotide polymorphisms or SNP (mutated base 1) at given loci of gene nucleotide seguence(s) (3) of an organism (including the human) which means the identification or detection of several homologous sequences (7,7') present in the said sequence(s) (3) differing by one base (1) and comprising the steps of :

- amplifying the homologous gene nucleotide sequences or portion thereof containing different loci into a first set(s) of target nucleotide sequences (homologous sequences 7 or 7'), and;
- amplifying a second fragment of the gene nucleotide sequence(s) being non mutated into a second set of target nucleotide sequences (8) ;
- putting into contact the first set(s) of target nucleotide sequences (7 or 7') upon an array of capture probes (9,9',10) (bound to a solid support (22) surface), the said array comprising a series of pair of capture probe, (9 and 9') having the same specific hybridization sequence complementary to a portion of the different target nucleotide sequences (7 and 7') but differing by a single base (20) complementary to the mutated base (1) present in the locus sequence to be characterized;
- putting into contact the second set of target nucleotide sequences (8) with a capture probe (10) having a specific hybridization sequence complementary to a portion of the said target nucleotide sequence (8) but differing by a single base (21) ;
- detecting and/or quantifying the signal value of hybridization of the first set of target nucleotide sequences (7 or 7') and the signal value of hybridization of the second set of target nucleotide sequences (8)and;
- determining the presence of the nucleotide base (1) at the different said loci wherein signal values of detection of the target sequence(s) (7 and/or 7') upon their corresponding capture probes (9 or 9') is higher or equal to a cut off signal value calculated from the signal value of detection of the second set of the target sequence (8) upon its corresponding specific mutated capture probe (10); wherein the capture sequences (9,9' or 10) have similar physical or chemical properties and;
- Determining the presence of single nucleotide polymorphisms (mutated base 1) in the different said loci.

In the method according to the invention, this detection is particularly adapted for the identification of multiple single nucleotide polymorphisms or multiple mutations (multiple SNPs) present at different gene locus. The method also provides tools and means to determine whether an organism is heterozygote (different alleles) or homozygote (same alleles) at a particular gene locus.

Preferably, said detection or characterization is obtained upon the same array. Furthermore, the step of comparing the signal value of hybridisation between the different sets of targets and their corresponding capture probes is preferably made upon the same array. The capture probes are preferably present at specific locations of a solid support 22 surface forming an array.

## Definition

Unless otherwise defined, all technical and scientific terms used herein have the same meaning as commonly understood by one person ordinary skilled in the art to which this invention belongs.

The terms "nucleotide sequence, array, target (and capture) nucleotide sequence, bind substantially, hybridizing specifically to, background, quantifying" are as described in WO97/27317, which is incorporated herein by way of reference.

The terms "nucleotide triphosphate, nucleotide, primer sequence" are those described in the European patent application EP1096024 incorporated herein by reference.

The term "gene" means fundamental physical and functional unit of heredity, which carries information from one generation to the next; a segment of DNA located in a specific site on a chromosome that encode a specific functional product. The DNA segment is composed of transcribed region and a regulatory sequence that makes transcription possible (regions preceding and following the coding DNA as well as introns between the exons).

The term "locus" means the position of the single nucleotide polymorphism (SNP) upon the sequence of the gene.

"Homologous sequences" mean nucleotide sequences having a percentage of nucleotides identical at corresponding positions which is higher than in purely random alignments. Two sequences are considered as homologous when they show between them a minimum of homology (or sequence identity) defined as the percentage of identical nucleotides found at each position compared to the total nucleotides, after the sequences have been optimally aligned taking into account additions or deletions (like gaps) in one of the two sequences to be compared. The degree of homology (or sequence identity) can vary a lot as homologous sequences may be homologous only in one part, a few parts or portions or all along their sequences. Nucleotide sequences differing by only one base are sequences highly homologous and qualified as single nucleotide polymorphisms (SNPs). The parts or portions of the sequences that are identical in both sequences are said conserved. Protein domains which present a conserved three dimensional structure are usually coded by homologous sequences and even often by a unique exon. The sequences showing a high degree of invariance in their sequences are said to be highly conserved and they present a high degree of homology.

Methods of alignment of sequences are based on local homology algorithms which have been computerised and are available as for example (but not limited to) Clustal®, (Intelligenetics, Mountain Views, California), or GAP®, BESTFIT®, FASTA® and TFASTA® (Wisconsin Genetics Software Package, Genetics Computer Group Madison, Wisconsin, USA) or Boxshade®.

The term "consensus sequence" is a sequence determined after alignment of the several homologous sequences to be considered (calculated as the base which is the most commonly found at each position in the compared, aligned, homologous sequences).

The consensus sequence represents a sort of «average» sequence which is as close as possible from all the compared sequences. For high homologous sequences or if the consensus sequence is long enough and the reaction conditions are not too stringent, it can bind to all the homologous sequences. This is especially useful for an amplification of homologous sequences with the same primers called, consensus primers. Experimentally, the consensus sequence calculated from the programs above can be adapted in order to obtain such property.

"Micro-arrays and arrays" mean solid supports on which single capture probes or capture probes species are immobilized in order to be able to bind to the given specific protein or target. The most common arrays are composed of single capture probes species being present in predetermined locations of a solid support being or not a substrate for their binding. The array is preferentially composed of spots of capture probes deposited at a given location on the surface or within the solid support or on the substrate covering the solid support. However, capture probes can be present on the solid support in various forms being but not limited to spots. One particular form of application of array is the presence of capture probes in wells having either one of several different capture probes per well and being part of the same support. Advantageously, arrays of capture probes are also provided on different supports as long as these different supports contain specific capture probes and may be distinguished from each other in order to be able to allow a quantification of a specific target sequence. This can be achieved by using a mixture of beads having particular features and being able to be recognized from each other in order to quantify the bound molecules.

The terms "capture probe" relate to molecules capable to specifically bind to a given polynucleotide or polypeptide. Preferably, polynucleotide binding is obtained through base pairing between two polynucleotides one being the immobilized capture probe or capture sequence and the other one being the target molecule (sequence) to be detected.

The term "single capture probe species" is a composition of related (poly)nucleotides for the detection of a given nucleotide sequence by base pairing hybridization. Polynucleotides are synthesized either chemically or enzymatically or purified from samples. However, the synthesis or purification is not always perfect and the capture probe can be slightly contaminated by other related molecules like shorter polynucleotides. The essential characteristic of one capture species for the invention is that the overall species can be used for capture of a given target molecule, preferably a given target nucleotide sequence.

The term "signal resulting from a specific binding at a specific location" means a detection and possibly a quantification of a single hybridization event between complementary nucleotide sequences at the specific localized area (location) of a fixed capture sequence of the solid support surface (or inside the solid support). A complementary hybridization can be detected and possibly quantified by a (fluorescent, colorimetric, etc.) label introduced in the sequence of the target sequence or at the extremity of the target sequence (preferably during the copy or amplification step) or by any of a number of means well known to those skilled in the art, such as detailed in WO 99/32660.

## Detailed description of the invention

The present invention provides unambiguous determination of the presence or the absence of a particular base (mutated base) in a locus of a genetic sequence by examining the signal obtained in a particular location of an array which serves to question (to detect) for the presence or not of the (mutated) base in a particular locus (SNPs).

The method allows a simultaneous detection of at least 3 and better 5 and still better 20 loci and accordingly, the solid support may contain at least 6 and better 10 and still better at least 40 different capture probes having a specific sequence complementary to the different target loci to be questioned and differing by only one base (SNPs detected).

The specific hybridization sequences present on the capture probes are complementary to the different targets that they correspond and they have similar chemical and physical properties. Preferably these specific sequences for on locus are identical for the target of a given locus except for the base to be questioned at the locus.

The gene nucleotide sequence(s) can be firstly extracted from a sample from the organism. Extraction means any kind of isolation of genetic material (mRNA, genomic DNA) from a sample by a physical or a chemical process. Assays on (micro)organisms extracted from a biological sample or from clinical sample or from cell culture, preferably requires an isolation of genomic DNA sequences. The different loci are either present on the same exon of a gene or on the same gene or on different genes which belong to the genome of the organism. The target sequences differing at one or at several given loci are homologous sequences.

The genetic amplification step used in the method according to the invention is performed by amplification protocols well known in the art, preferably by a method selected from the group consisting of PCR, RT-PCR, LCR, CPT, NASBA, ICR or Avalanche DNA techniques.

The nucleotide sequence of a gene is amplified using at least one primer pair (i.e. a pair of two different primers). However, several primer pairs are either used for amplifying the different specific nucleotide sequences of a gene, these sequences being preferably different exons, or used for amplifying different genes or different parts of a cell genome.

Preferably, each amplified target sequence comprises several loci. All these loci of the target are then amplified with the same primer pair being consensus primers for an amplification of all these loci, but each locus is detected on specific capture probes.

Therefore, the array contains capture probes specific for one or more loci for hybridization with target nucleotide sequence(s) comprising the mutated bases (1) to be detected in each locus, the different mutated bases being located in the same exon or in different exons originating from the same gene or from different genes, preferably present in the same nucleotide sequence (3). The amplification step of these several exons is preferably obtained with different primer pairs, each primer pair being specific for one exon. Amplification of several exons is preferably performed in the same conditions for all exons (i.e; in the same reaction vessel or in different vessels) and are then pooled together for hybridization on a microarray. They constitute the first set of targets (7 or 7').

The second set of target nucleotide sequences (8) is amplified preferably with the same primers used for the first set of target sequences (7 or 7'). In this preferred embodiment, each target sequence amplified by one primer pair (5, 6'), also contains a sequence hybridized on the second set of capture probe (10). However, the inventors have found that the requirement for the presence of the second set of target sequences on the same amplified sequence is not needed as long as that the amplifications of both target sequences using different primer pairs (5, 5' and 6, 6') are similar. The similarity of the results is obtained by comparing the efficiency of hybridization of both amplified sequences (7,7', 8) on the corresponding capture probes (9,9',10) having complementary sequences. The signals have to be identical or differing by a factor lower than 3 and preferably lower than 2.

In the next step of the method of the invention, amplified target nucleotide sequences (7 or 7') of the first step (amplicons) are contacted with an array under conditions allowing hybridization of the target amplified sequences (7,7') to their complementary specific capture nucleotide sequences (9,9')(capture probes) present on the array. The amplicons obtained in the first step are hybridized on the same or on different arrays. In a preferred embodiment, these amplified sequences are processed (e.g. by fragmentation) prior to hybridization on the array. Fragmentation of double stranded DNA is obtained using enzymatic cleavage (DNase treatment) or chemical cleavage, preferably (depurination using HCL followed by heat denaturation as described in the patent application US 2005/0095635). Fragmentation of ribonucleotide sequences is obtained by heating in the presence of magnesium ions or in the presence of KOH as described respectively in WO97/10365 and WO98/28444.

Parts (or portions) of the gene or genome sequence (loci) having possible mutations to be detected can be firstly amplified by PCR and the resulting amplicons are fragmented by DNAse treatment (Grimm et al. 2004, Journal of Clinical Microbioloy, 42, 3766-3774). In the preferred embodiment, the resulting amplicon fragments are between 30 and 70 bases long. The distribution of the fragments size obtained after fragmentation of the amplicons is advantageously checked by analysis by capillary electrophoresis (Bioanalyser, Agilent) and the average size distribution of the pieces is preferably comprised between 30 and 70 bases long.

The specific part (or portion) of the capture probes (nucleotide sequences) complementary to the target nucleotide sequence is comprised between about 10 and about 50 bases, preferably between about 15 and about 40 bases and more preferably between 18 and 24 bases. These bases are preferably assigned as a continuous sequence located at or near the extremity of the capture probes (nucleotide sequences). This sequence is considered as a specific sequence for the detection of the target nucleotide sequence.

Preferably, the melting temperature (Tm) of the specific nucleotide sequences of the capture probes (able to bind to their corresponding target nucleotide sequences) is comprised between 55 and 75°C and preferably between 62 and 68°C. The Tm of small sequences are easily calculated from the simplified equation of Tm (°C) = 4 (G+C) + 2 (A+T).

Preferably, the hybridization step between capture and target sequence is carried out under stringent conditions. Temperatures of hybridization is preferably between 1 and 10 °C and even preferably 1 to 4°C lower than the Tm of the specific sequence of the capture probes. The hybridization temperature can be adapted according to the presence of molecules in the hybridization solution affecting the hybridization temperature such as the DMSO or the formamide.

Hybridization of the target sequences is performed at a temperature giving the best signal together with the lowest cross-reaction of the target on capture probes differing by one base. The optimized temperature is related on the stringency of the solution which depends on the solution composition and mainly on the salt concentration. In a preferred embodiment, the hybridization solution is composed of phosphate buffer at ph 7.4 of a molarity comprised between 0.4M to 0.8M and the hybridization temperature differs from the Tm of the specific sequences of the capture probes (9, 9 and 10) by no more than 5°C and preferably by no more than 2°C. In a particular embodiment the hybridization temperature is 60°C.

On the array, capture probes are arranged at predetermined locations at a density of at least 4, 10, 16, 20, 50, 100, 1000, 4000, 10 000 or more, different capture probes/cm$^2$ insoluble solid support surface. The capture probes are advantageously covalently attached to the surface of the solid support (preferably a non porous solid support surface) by one of their extremities, preferably by their 5' end. The sensitivity may be further increased by spotting capture probes on the solid support surface by a robot at high density according to an array. The amount of capture probes spotted on the array is preferably comprised between about 0.01 to about 5 picomoles of sequence equivalent/cm$^2$ of solid support surface.

The invention is also related to a kit for the detection of a nucleotide base at a given locus of a gene nucleotide sequence of an organism (for characterizing the presence of a single nucleotide polymorphism) among at least 2 possible nucleotide bases and which incorporates media or devices for performing the method according to the invention. The kit is preferably included in an automatic apparatus (that performs most of the steps of the present invention automatically), such as a high throughput screening apparatus. The kit or apparatus is adapted for performing all the steps or only several specific steps of the method according to the invention

This kit comprises:

- an insoluble solid support (22) surface, preferably a non porous solid support surface comprising :

  - (a) at least two capture probes (9,9') bound at a particular location of the insoluble solid support (22) surface, the capture probes (9,9') having a specific sequence complementary to a portion (2) of a target nucleotide sequence (7 or 7') comprising a first locus and wherein each capture probe (9) differs from the other capture probe (9') by a single base (20) at the locus site, and
  - (b) at least one further capture probe (10) being located external to the first locus and having similar physical and chemical properties as the other two capture probes (9,9') and having a complementary sequence to another portion (4) of the target nucleotide sequence (8), except for one base (21) not being complementary (the target and capture nucleotide sequences are complementary sequences but this complementarity differs by one base) and,

- a carrier means embedded computerized code for determining (characterizing/or detecting) the presence of the nucleotide base (1) at a given locus wherein a signal value of detection of the target sequence (7 or 7') on their specific capture probe (9 or 9') higher or equal to a cut off signal value calculated from the signal value of detection of the target sequence (8) on their specific mutated capture probe (10) gives a positive result identifying (detecting the presence of) the nucleotide base (1).

In a further step, the carrier means embedded computerized code of the kit determines that the organism is heterozygote at given locus when the signal values of detection of the target nucleotide sequences (7 and 7') upon mutated and non mutated capture probes (9 and 9') are both positive and said signal values possibly comply with the condition that the index calculated (CI) according to the formula: $CI = \dfrac{(N9-N9')}{(N9+N9')/2}$ is lower than 1, preferably lower than 0.7 and even preferably lower than 0.5; with N being the average signal value of detection upon mutated (9) or non mutated (9') capture probes.

The solid support surface contains at least 10 different capture probes presented as an array targeting 5 different loci of gene(s) and better at least 40 different capture probes targeting at least 20 different loci.

In a preferred embodiment, the capture probes comprises a specific sequence for the binding to the target nucleotide sequence linked to the solid support (22) surface by a spacer (or linker) having a physical length of at least about 6.8 nm, equivalent to the distance of at least about 20 base pair long nucleotides in double helix form.

In a preferred embodiment, the spacer (or linker) is a polynucleotide being at least about 20 nucleotides long at least about 50 or about 70 nucleotides and preferably at least about 90 nucleotides long. The spacer (or linker) is a given nucleotide sequence being homologous to none of the genome sequence (when using an identity of at least 10 and better 5 consecutive bases). To avoid non specific hybridization, there will be no more than around 15 consecutive complementary base pair bindings between a target polynucleotide (or nucleotide) sequence and the spacer, preferably there will be less than 10 such pairings possible, more preferably less than 5. As such, the nucleotide sequence of the spacer will contain, preferably less than 15 bases and more preferably, less than 10 and still more preferably less than 5 contiguous bases complementary to the target nucleotide sequences to be detected. The determination of possible consecutive sequences is easily done by comparison of the sequences to molecular database as provided by Genbank and using software such as nucleotide-nucleotide BLAST(blastn)(http://www.ncbi.nlm.nih.gov/BLAST).

The total length of the capture probes (nucleotide sequences) including the spacer is comprised between about 30 and about 300 bases, preferably between about 35 and about 200 bases, more preferably between about 39 and about 120 bases.

In another preferred embodiment of the invention, capture probes (nucleotide sequences) are chemically synthesised oligonucleotide sequences of about 100 bases, which may e.g. be easily performed on programmed automatic synthesizer. Such sequences can bear a functionalized group for covalent attachment upon the support, at high concentrations. Longer capture nucleotide sequences are preferably synthesised by PCR amplification of a sequence incorporated into a plasmid containing the specific part (or portion) of the capture nucleotide sequence and the non specific part (or portion) (spacer).

Chemical and physical properties means features of the external capture probes (10) which give an efficiency of hybridization similar to the specific capture probe (9 or 9') for the mutations to be detected.

The chemical and physical properties comprise: the specific part of the nucleotide sequence (length, Tm), the percentage of GC content and the length of the spacer.

In a preferred embodiment, the capture probe (10) external to the locus has the same features as the specific capture probes (9, 9').

In a preferred embodiment, similar physical or chemical properties of nucleotide sequences means that the specific nucleotide sequences of the capture probes (able to bind to the target nucleotide sequences) have a Tm comprised between about 55 and about 75°C and preferably between about 62 and about 68°C.

In a further preferred embodiment, capture probes (9, 9') differing between them by one base have, in their specific nucleotide sequences (able to bind specifically the target nucleotide sequences) have a GC content comprised between about 40 and about 70% and preferably between about 45 and about 60%.

Furthermore, the inventors have found unexpectedly that a better discrimination of the SNP present in a locus of a gene is obtained when the nucleotide base to be detected is located at a distance of about 4 to about 10 bases and better 4 to 6 bases from one extremity of the target specific part of the bound capture probe.

The spacer (linker) is preferably located at the 5' extremity of the capture probe being fixed to the surface of the solid support by a covalent link present at the 5' end or nearby. The specific nucleotide sequence for the binding to the target nucleotide sequence is preferably located at 3' end of the capture probes (free extremity not bound to the support) at 1 to 23 nucleotides from the end.

The capture probes (9 or 9' and 10) preferably differ by one base located at 4 to 10 and preferably at 4 to 6 bases from the (free) 3' end of the target specific part (portion) of the bound capture probe.

The array may contain specific capture nucleotide sequences for each base of a specific locus to be detected. The bases to be detected are present within one or several exons of the same gene nucleotide sequence or from different gene nucleotide sequences.

In a preferred example, the array contains specific capture probes (9, 9', 10) for the detection of SNP in human Cytochromes P450 2C9, 2C19 and 2D6. Cytochromes p450 2C9 and 2D6 are preferably detected upon an array of capture probes (9,9',10) containing specific, as provided in table 1. Cytochrome P450 for mutations 2C9*1,2 and 1,8 and for 2C19*1,2 and 1,3 are also preferably added on the array together with the mutations provided in table 1.

The array may contain specific capture probes for the detection of several SNP in one gene nucleotide sequence, or the array may contain specific capture probes for the detection of several SNP in different gene nucleotide sequences.

The target nucleotide sequences are labelled during the amplification step. The labelled associated detections are numerous. A review of the different labelling molecules is given in W0 97/27317. They are obtained using either already labelled primer or by incorporation of labelled nucleotides during the amplification step. The most frequently used and preferred labels are fluorochromes like Cy3, Cy5 and Cy7 suitable for analysing an array by using commercially available array scanners (General Scanning, Genetic Microsystem, ...).

Radioactive labelling, cold labelling or indirect labelling with small molecules recognised thereafter by specific ligands (streptavidin or antibodies) are common methods. The resulting signal of target fixation on the array is either fluorescent, colorimetric, diffusion, electroluminescent, bio- or chemiluminescent, magnetic, electric like impedometric or voltametric (US-A-5,312,527).

A preferred method is based upon the use of the gold labelling of the bound target in order to obtain resonance light scattering (RLS) detection or silver staining which is then easily detected and quantified by a scanner. Gold particles of 10-30 nm are required for silver amplification while particles of 40-80 nm are required for direct detection of gold particles by RLS or by Photothermal Heterodyne Imaging.

In a preferred method, gold particles of 10-30 nm are amplified by silver enhancement preferably using the silverquant analysis platform including the Silverquant kit for detection, the Silverquant Scanner for slide scanning and Silverqaunt Analysis software for image quantification and data analysis (Eppendorf, Germany). Due to the non linear detection of the presence of silver, the data analysis requires a linearization of data before data processing. The data are then processed according to the invention. An algorithm of curve fitting is applied to a positive detection curve spotted on the array. Then each spot signal is linearized in 'concentration units' using the fitting curve.

Quantification has to take into account not only the hybridisation yield and detection scale on the array but also the extraction, the amplification (or copying) and the labelling steps.

The solid support according to the invention is made with materials selected from the group consisting of gel layers, glasses, electronic devices, silicon or plastic support, polymers, compact discs, metallic supports or a mixture thereof (see EP 0 535 242, US 5,736,257, WO99/35499, US 5,552,270, etc). Advantageously, the solid support is a single glass slide which may comprise additional means (barcodes, markers, etc.) or media for improving the method according to the invention.

In the last step of the method of the invention, results are considered positive or negative according to the fact that the signal intensity is higher or lower than a determined cut off value.

The cut off signal values are preferably taken as detected signal values of the external capture probe (10) targeting a corresponding target sequence (8) and multiplied by a factor.

In a preferred embodiment, the cut off signal value is the signal value of detection of the second set of target nucleotide sequences (8) upon its corresponding specific mutated capture probe (10) multiplied by a factor between 2 and 5 and preferably between 3 and 4, more preferably the factor 4.

In another preferred embodiment, the cut off signal value is 4 times the signal obtained with the capture probes (10) external to the detected locus, being preferably the mean value of replicated spots.

Preferably, the cut off signal value is calculated from an average signal value of detection of at least two different sets of target nucleotide sequences (8) upon at least two different complementary specific mutated capture probes (10).

The signal values of the capture probes (7 or 7') targeting specific target loci are considered for a calculation of the cut off signal value, as long as they have the features of external capture probes (10) including differing from the target by one base.

Preferably, the cut off signal value is calculated from an average signal value of detection of at least two different sets of target nucleotide sequences (7 or 7') upon at least two different complementary specific mutated capture probes (9 or 9').

The method of the invention is also suited for detection of SNP in organism being heterozygote at a given locus. In a preferred embodiment, the organism is considered as heterozygote at given loci when the signal values of detection of the target nucleotide sequences (7 and 7') upon mutated and non mutated capture probes (9 and 9') are both positive.

The organism can also be considered as heterozygote at given loci when the signal values of detection of the target nucleotide sequences (7 and 7') upon mutated and non mutated capture probes (9 and 9') are both positive and furthermore that said signal values comply with the condition that the index calculated (CI) according to the formula :

$$CI = \frac{(N9 - N9')}{(N9 + N9')/2}$$

is lower than 1;
with N being the average signal value of detection upon mutated (9) or non mutated (9') capture probes. In a still preferred embodiment the CI is lower than 0.7 and even better lower than 0.5.

In a preferred embodiment, the organism is considered as homozygote at a given locus when only one of the signal values of detection of the target nucleotide sequences (7 or 7') upon mutated and non mutated capture probes (9 or 9') is positive.

Advantageously, the array also contains capture probes for the specific detection of the specifically amplified target gene(s) and being located outside the locus of interest. Such capture probes are preferably added for confirmation of the identification of gene(s) or for discrimination between two genes being very homologous. The capture probe is used as positive control of the PCR and hybridization.

Advantageously, the array also contains spots with various concentrations (i.e. 4) of labelled capture probes. These labelled capture probes are spotted from known concentrations solutions and their signals allow the conversion of the results of hybridisation into absolute amounts. They also allow testing for the reproducibility of the detection.

In one embodiment, the solid support (biochip) is inserted in a support connected in and out pipes and handled by an automatic machine controlling liquid solution as being developed in the microfluidic technology. By being inserted into such a microlaboratory system, it can be incubated, heated, washed and labelled by automates, even for previous steps (like extraction of DNA, amplification by PCR) or the following step (labelling and detection). All these steps can be performed upon the same solid support.

Preferably, the kit of the invention comprises at least an insoluble solid support upon which are bound some capture probes (preferably bound to the surface of the solid support by a direct covalent link or by the intermediate of a spacer) according to an array with a density of at least 4, preferably at least 10, 16, 20, 50, 100, 1000, 4000, 10 000 or more, different capture probes(s)/cm$^2$ insoluble solid support surface, said capture probes having advantageously a length comprised between about 30 and about 300 bases (including the spacer) and containing a sequence of about 10 to about 50 bases, said sequence being specific for the target (which means that said bases of said sequence are able to form a binding with their complementary bases upon the sequence of the target by complementary hybridisation).

## Brief description of the drawings

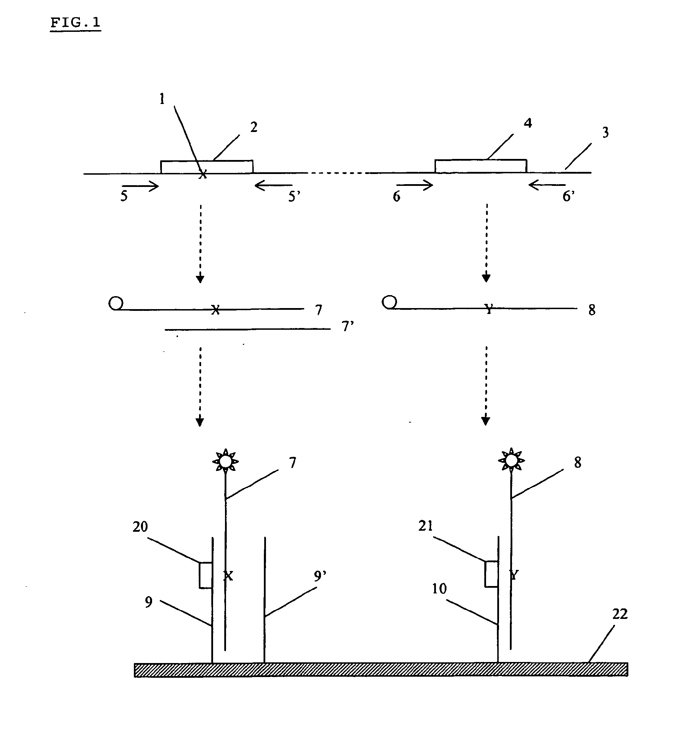

Figure 1 is a schematic representation of a preferred method of the invention for the detection of a SNP (mutated base 1 represented by X) at given locus of gene nucleotide sequence (3) of an organism. A portion of the gene nucleotide sequence is amplified by PCR using either a single primer pair (5, 6') or different primer pairs (5, 5' and 6, 6') into a first set of target nucleotide sequences (7 or 7') and a second set of target nucleotide sequence (8) which are both labelled (circle). The amplicons, after labelling, are then contacted with an array of capture probes immobilized on a solid support (22) comprising : (a) a pair of capture probes (9 and 9') having the same specific hybridization sequence complementary to a portion (2) of the target nucleotide sequence (7 and 7') but differing by a single base (20) corresponding to the mutated base (1) present at the locus to be characterized; (b) a capture probe (10) having a specific hybridization sequence complementary to a portion (4) of the target nucleotide sequence (8) but differing by single base (21) at position Y. The presence of SNP is determined if the signal value of detection of the target sequence (7 and/or 7') upon their corresponding capture probes (9 or 9') is higher or equal to a cut off signal value of detection of the target sequence (8) upon its corresponding specific mutated capture probe (10). All capture probes have similar physical and chemical properties.

Table 1 presents the list of probes for SNP detection on *CYP2D6* and *CYP2C9* human genes. Sequence, melting temperature (Tm), GC percentage (% GC) and length of each probe are detailed. The substituted nucleotides are underlined; the points represent deleted nucleotides.

Table 2 presents the result of SNP detection of a sample CYP2D6*1/*4 combined with CYP2C9*1/*3. The sample have been hybridized 5 times (n=5).

Table 3 presents the result of SNP detection of a sample CYP2C9*9. The sample have been hybridized 3 times (n=3).

## Examples

### *Example 1: Detection of 2 mutations in CYP2C9 (exon 3)*

The *CYP2C9* gene containing 2 main mutations in the exon 3 is amplified by PCR using the following primers:

| | | |
|---|---|---|
| *MP2C19 06 :* | 5' AGCAATGGAAAGARATGGAAGGAG 3' | (SEQ ID NO:1). |
| *MP2C9 07 :* | 5' GAAACCCCAGAGAAGTCAGTGAG 3' | (SEQ ID NO:2) |

The PCR was performed on genomic DNA extracted from blood sample with the kit Quiaamp DNA Blood mini (Qiagen, venlo, Netherlands) in a final volume of 50 μl containing: 3 mM MgCl$_2$, 10 mM Tris pH 8.4, 50 mM KCl, 125 μM of each primer, 200 μM of dATP, dCTP and dGTP (Roche), 150 μM of dTTP, 50 μM of dUTP, 10 μM of biotin-11-dATP (PerkinElmer), 10 μM of biotin-11-dCTP (PerkinElmer), 2.5 U of Taq DNA polymerase Ultratools (Labsystems), 25 ng of genomic DNA. Samples were first denatured at 94 °C for 5 min. Then 40 cycles of amplification were performed consisting of 30 sec at 94 °C, 1 min at 63 °C and 1 min at 72 °C and a final extension step of 10 min at 72 °C. Water controls were used as negative controls of the amplification. The expected size is 622 bp.

*Capture nucleotide sequence immobilisation*

The protocol described in WO02/18288 (example 1 and 2) was followed for obtaining aldehyde derivatised glass slide (diaglass slide). A list of capture probes for SNP detection on *CYP2D6* and *CYP2C9* human genes are presented in table 1. The aminated capture nucleotide sequences were spotted on diaglass slide according to the protocol described in WO01/77372 (example 1) at a concentration of 3000 nM.

*Fragmentation*

After the PCR, the amplified sequences (amplicons) are purified, quantified. The amplified DNA are fragmented with a DNAse (Promega, Madison, USA) at room temperature for 5 min. The reaction are stopped by the addition of 3 mM EGTA and incubation at 65°C for 10 min (Grimm et al. 2004, Journal of Clinical Microbioloy, 42, 3766-3774). The DNAse cuts the amplicons randomly giving rise to fragments of approximately 50 bp. The efficiency of the fragmentation is checked using a DNA 1000 LabChip® (Agilent technologies, Germany).

*Hybridisation*

A volume of 10 $\mu$l of NaOH 0.175 N was added at 10 $\mu$l of fragmentated amplicons, 5 $\mu$l of positive hybridisation control and 10 $\mu$l of distilled water. The mix was incubated at room temperature for 5 min. A volume of 35 $\mu$l of Genomic Hybribuffer (Eppendorf, Germany) was finally added and the solution was loaded on the array framed by a hybridisation chamber. The chamber was closed with a coverslip. The hybridisation was carried out at 60° for 2 h. Samples were washed 4 times with Unibuffer (Eppendorf, Germany).

*Colorimetric detection*

The glass samples were incubated 45 min at room temperature with colloidal gold-conjugated IgG Anti-biotin 1000 x diluted in blocking buffer. After 5 washes with washing buffer, the presence of gold served for catalysis of silver reduction using the Silverquant kit (Eppendorf, Hamburg, Germany). The slides were incubated 1 times 5 min with the revelation mixture of Silverquant A and B solutions, then rinsed with water, dried and analysed using the Silverquant scanner (Eppendorf, Hamburg, Germany). Each slide were then quantified by the silverquant Analysis software. A curve fitting algorithm was used to linearize the signal intensities obtained with silverquant detection method. The algorithm used a positive detection curve spotted on the array.

*Fluorescence detection*

The glass samples were incubated 45 min at room temperature with the Cy3-conjugated IgG Anti-biotin (Jackson Immuno Research Laboratories, Inc #200-162-096) diluted 1/1000 X Conjugate-Cy3 in the blocking buffer and protected from light. After washing the slides were dried before being stored at room temperature. The detection was performed in the laser confocal scanner "ScanArray"™ (Packard, USA) Each slide was then quantified by the Imagene quantification software (Biodiscovery).

*Data analysis*

The signals are compared to a threshold capture probe which is the mutated external Hybridization probe. If the signal is higher than the threshold probe multiplied by a factor 4, the result is considered as positive. Inversely, if the signal is lower, the result is considered as negative.

**Example 2: Detection of multiple mutations in CYP2D6 and CYP2C9 (exons 5 and 7)**

The *CYP2D6* gene containing 3 main mutations and the *CYP2C9* gene containing 6 main mutations in the exons 5 and 7 were amplified by PCR using the following primers:

| | | |
|---|---|---|
| *MP2D6 01* : | 5' CGCTTCTCCGTGTCCACCTTGC 3' | (SEQ ID NO:3) |
| *MP2D6 02 :* | 5' GCCACTCTCACCTTCTCCATCTC 3' | (SEQ ID NO:4) |
| *MP2C9 06:* | 5' GCTTTGTACTATCAATCAGGTTGTC 3' | (SEQ ID NO:5) |
| *MP2C9 02:* | 5' CACAAATTCACAAGCAGTCACATAAC 3' | (SEQ ID NO:6) |
| *MP2C9 03:* | 5' CTAAAGTCCAGGAAGAGATTGAACG 3' | (SEQ ID NO:7) |
| *MP2C9 04:* | 5' CAGAGTGTTGATTTGACAAGATTTTAC 3' | (SEQ ID NO:8) |

The expected sizes of the amplicons were 1014 bp for *CYP2D6,* 626 bp for *CYP2C9* exon 5 and 1114 bp for *CYP2C9* exon 7.

PCRs, fragmentations, hybridisations, detection were performed as described in the example 1 using fluorescence detection. The specific parts of the capture probes for hybridization of the targets are presented in table 1. This table comprises capture probes for the detection of 9 mutations as well as external capture probes (10) of the invention (2D6 and 2C9 external probes) corresponding to each amplified exon. The array also contains control probes which are the non mutated external probes of the invention (2D6 and 2C9 control probes). The analysis was performed on several clinical samples and the data presented in table 2 and 3. The results were analysed as proposed in the invention and the final results were compared with the determination of the sample mutations by sequencing. There was 100% correlation of the results.

**Table 1:** List of probes for SNP detection on *CYP2D6* and *CYP2C9* genes (SEQ ID NOs: 9-30). Sequence, melting temperature (Tm), GC percentage (% GC) and length of each probe are detailed. The substituted nucleotides are underlined; the points represent deleted nucleotides.

| Mutation | Sequence | Tm (°C) | %GC | Length |
|---|---|---|---|---|
| 2D6*1,3 | CTGCTAACTGAGCACAGGATG | 64 | 52,4 | 21 |
| 2D6*3 | CTGCTAACTGAGCAC.GGATGA | 64 | 52,4 | 21 |
| 2D6*1,4 | GCGAAAGGGGCGTCCTGGG | 66 | 73,7 | 19 |
| 2D6*4 | GCGAAAGGGGCGTCTTGGG | 64 | 68,4 | 19 |
| 2D6*1,6 | CCTCGGTCACCCACTGCTC | 64 | 68,4 | 19 |
| 2D6*6 | TCCTCGGTCACCC.CTGCTC | 64 | 68,4 | 19 |
| 2D6 control probe | GTTGGCGAAGGCGGCACAAA | 64 | 60 | 20 |
| 2D6 external probe | GTTGGCGAAGGCGGAACAAAG | 66 | 57,1 | 21 |
| 2C9*1,9 | TGTCCATTGATTCTTGGTGTTC | 62 | 40,9 | 22 |
| 2C9*9 | GTCCATTGATTCTTGGCGTTC | 62 | 47,6 | 21 |
| 2C9*1,10 | CTTCCTGATGAAAATGGAGAAGG | 66 | 43,5 | 23 |
| 2C9*10 | CTTCCTGATGAAAATGGGGAAGG | 68 | 47,8 | 23 |
| 2C9*1,3=*1,4 | GGTGGGGAGAAGGTCAATGTA | 64 | 52,4 | 21 |
| 2C9*3 | GGTGGGGAGAAGGTCAAGGTA | 66 | 57,1 | 21 |
| 2C9*1,4 | GGTGGGGAGAAGGTCAATGTA | 64 | 52,4 | 21 |
| 2C9*4 | GGTGGGGAGAAGGTCAGTGTA | 66 | 57,1 | 21 |
| 2C9*1,5 | GCTGGTGGGGAGAAGGTCAA | 64 | 60 | 20 |
| 2C9*5 | GCTGGTGGGGAGAAGCTCAA | 64 | 60 | 20 |
| 2C9*1,11 | ATGCAGGGGCTCCGGTTTCT | 64 | 60 | 20 |
| 2C9*11 | CATGCAGGGGCTCCAGTTTC | 64 | 60 | 20 |
| 2C9 control probe | GCAGGCTGGTGGGGAGAAG | 64 | 68,4 | 19 |
| 2C9 external probe | GCAGGCTGGTGGGAAGAAG | 62 | 63,2 | 19 |

**Table 2:** Sample CYP2D6*1/*4 combined with CYP2C9*1/*3. The sample have been hybridized 5 times (n=5).

| Mutation | Mean values n=5 | Standard Deviation | Results Invention | Results Sequencing |
|---|---|---|---|---|
| 2D6*1,3 | 14769 | 2799 | + | + |
| 2D6*3 | 130 | 130 | - | - |
| 2D6*1,4 | 14025 | 2926 | + | + |
| 2D6*4 | 15354 | 2562 | + | + |
| 2D6*1,6 | 51862 | 4289 | + | + |
| 2D6*6 | 1164 | 174 | - | - |
| 2D6 control probe | 20273 | 3595 | + | + |
| 2D6 external probe | 729 | 221 | - | - |
| 2C9*1,9 | 16664 | 2731 | + | + |
| 2C9*9 | 681 | 111 | - | - |

(continued)

| Mutation | Mean values n=5 | Standard Deviation | Results Invention | Results Sequencing |
|---|---|---|---|---|
| 2C9*1,10 | 20239 | 2929 | + | + |
| 2C9*10 | 1090 | 140 | - | - |
| 2C9*1,3=*1,4 | 33802 | 4299 | + | + |
| 2C9*3 | 28589 | 2903 | + | + |
| 2C9*1,4 | 33803 | 4299 | + | + |
| 2C9*4 | 2342 | 247 | - | - |
| 2C9*1,5 | 53000 | 9404 | + | + |
| 2C9*5 | 368 | 111 | - | - |
| 2C9*1,11 | 65061 | 165 | + | + |
| 2C9*11 | 1990 | 224 | - | - |
| 2C9 control probe | 65156 | 83 | + | + |
| 2C9 external probe | 818 | 183 | - | - |

**Table 3:** Sample CYP2C9*9. The sample have been hybridized 3 times (n=3).

| Mutation | Mean values n=3 | Standard Deviation | Results Invention | Results Sequencing |
|---|---|---|---|---|
| 2D6*1,3 | 16055 | 784 | + | + |
| 2D6*3 | 795 | 57 | - | - |
| 2D6*1,4 | 29935 | 334 | + | + |
| 2D6*4 | 2566 | 196 | - | - |
| 2D6*1,6 | 44038 | 2992 | + | + |
| 2D6*6 | 1012 | 115 | - | - |
| 2D6 control probe | 33486 | 1150 | + | + |
| 2D6 external probe | 1285 | 51 | - | - |
| 2C9*1,9 | 437 | 15 | - | - |
| 2C9*9 | 13084 | 556 | + | + |
| 2C9*1,10 | 15250 | 1015 | + | + |
| 2C9*10 | 731 | 87 | - | - |
| 2C9*1,3=*1,4 | 41655 | 2258 | + | + |
| 2C9*3 | 1953 | 73 | - | - |
| 2C9*1,4 | 41655 | 2258 | + | + |
| 2C9*4 | 2449 | 171 | - | - |
| 2C9*1,5 | 53196 | 4270 | + | + |
| 2C9*5 | 253 | 14 | - | - |
| 2C9*1,11 | 65048 | 65 | + | + |
| 2C9*11 | 1619 | 142 | - | - |
| 2C9 control probe | 65172 | 13 | + | + |
| 2C9 external probe | 617 | 80 | - | - |

## Claims

1. A method to determine the presence or absence of at least 3 single nucleotide polymorphisms or SNP(s) (mutated base 1) at given loci of gene nucleotide sequence(s) (3) of an organism and comprising the steps of:

    - amplifying the homologous gene nucleotide sequence(s) or portion thereof containing different loci into a first set(s) of target nucleotide sequences (homologous sequences 7 or 7'), and;
    - amplifying a second fragment of the gene nucleotide sequence(s) being non mutated into a second set of target nucleotide sequences (8) ;
    - putting into contact the first set(s) of target nucleotide sequences (7 or 7') upon an array of capture probes (9, 9', 10) bound to the surface of a solid support (22), the said array comprising a series of pair of capture probe, (9 and 9') having the same specific hybridization sequence complementary to a portion (2) of the different target nucleotide sequences (7 and 7'), but differing by a single base (20) complementary to the mutated base (1) present in the locus sequence to be **characterized**;

- putting into contact the second set of target nucleotide sequence (8) with a complementary capture probe (10) having a specific hybridization sequence complementary to a portion (4) of said target nucleotide sequence (8), but differing by a single base (21);

- detecting and/or quantifying the signal value of hybridization of the first set of target nucleotide sequences (7 or 7') and the signal value of hybridization of the second set of target nucleotide sequences (8);

- determining the presence of the nucleotide base (1) at the different said loci wherein signal values of detection of the target sequence(s) (7 and/or 7') upon their corresponding capture probes (9 or 9') are higher or equal to a cut off signal value calculated from the signal value of detection of the second set of the target sequence (8) upon its corresponding specific mutated capture probe (10); wherein the different capture probes (9,9' and 10) have similar physical and chemical properties, and;

- determining the presence of single nucleotide polymorphisms (mutated base 1) in the different said loci.

2. The method according to claim 1, wherein similar physical and chemical properties of the capture probes (9,9' and 10) have a GC content comprised between about 40 and about 70%.

3. The method according to claim 1 or 2, wherein the length of the capture probe (9,9' and 10) is comprised between about 15 and about 40 bases.

4. The method according to any of the preceding claims, wherein the capture probes have a Tm comprised between about 55 and about 75°C.

5. The method according to any of the preceding claims, wherein the hybridization temperature is preferably between 1 and 10 °C lower than the melting temperature (Tm) of the specific sequence of the capture probes (9, 9' and 10).

6. The method according to any of the preceding claims, wherein the capture probes are covalently attached to the surface of the solid support by their 5' end.

7. The method according to any of the preceding claims, wherein the capture probes (9 or 9' and 10) differs by one base located at a distance of about 4 to about 10 bases from the 3' end of the target specific part of the bound capture probe.

8. The method according to any of the preceding claims, wherein the cut off signal value is the signal value of detection of the second set of target nucleotide sequences (8) upon its corresponding specific mutated capture probe (10) multiplied by a factor between 2 and 5.

9. The method according to claim 8, wherein the cut off signal value is calculated from an average signal value of detection of at least two different sets of target nucleotide sequences (8) upon at least two different complementary specific mutated capture probes (10).

10. The method according to claim 8 or 9, wherein the cut off signal value is calculated from an average signal value of detection of at least two different sets of target nucleotide sequences (7 or 7') upon at least two different complementary specific mutated capture probes (9 or 9').

11. The method according to any of the preceding claims, wherein the organism is considered as heterozygote at given loci when the signal values of detection of the target nucleotide sequences (7 and 7') upon mutated and non mutated capture probes (9 and 9') are both positive.

12. The method according to claim 11, wherein the organism is considered as heterozygote at given loci when the signal values of detection of the target nucleotide sequences (7 and 7') upon mutated and non mutated capture probes (9 and 9') are both positive and furthermore that said signal values comply with the condition that the index calculated according to the formula :

$$CI = \frac{(N9 - N9')}{(N9 + N9')/2}$$

is lower than 1;

wherein N is the average signal value of detection upon mutated (9) or non mutated (9') capture probes.

13. The method according to claim 12, wherein the CI is lower than 0.7.

14. The method according to any of the preceding claims, wherein the first and second sets of target nucleotide sequences (7 or 7' and 8) are obtained by amplification with the same primer pair (5, 6').

15. The method according to any of claims 1 to 13, wherein the first and second sets of target nucleotide sequences (7 or 7' and 8) obtained by amplification with different primer pair (5, 5' and 6, 6') give detection signal on their specific capture probes which are identical or differing by a factor lower than 3.

16. The method according to any of the preceding claims, for the detection of SNP in human Cytochromes P450 2C9, 2C19 and 2D6.

17. The method according to claim 16, wherein the cytochromes p450, 2C9 and 2D6 are detected upon an array of capture probes (9,9',10) containing specific sequences as provided in table 1.

18. The method according to any of the preceding claims, wherein the target nucleotide sequences are labelled during the amplification.

19. The method according to any of the preceding claims, wherein the target nucleotide sequences are fragmented after the amplification.

20. The method according to claim 19, wherein the target nucleotide sequences are fragmented by DNase treatment.

21. The method according to any of the preceding claims, wherein the capture probes comprises a specific sequence for the binding to the target nucleotide sequence linked to the solid support (22) surface by a spacer.

22. The method according to claim 21, wherein the spacer is a polynucleotide of at least about 20 nucleotides long.

23. The method according to claim 21 or 22, wherein the spacer is a polynucleotide of at least about 90 nucleotides long.

24. A kit for the detection of a nucleotide base (1) at a given locus of a gene nucleotide sequence (3) of an organism comprising:

   - an insoluble solid support (22) surface, preferably a non porous solid support surface comprising:

      (a) at least two capture probes (9,9') bound at particular locations of said insoluble solid support (22) surface, said capture probes (9,9') having a specific sequence complementary to a portion (2) of a target nucleotide sequence (7 or 7') comprising a first locus and wherein each capture probe (9) differs from the other capture probe (9') by a single base (20) at the locus site;
      (b) at least one further capture probe (10) being located external to the first locus and having similar physical and chemical properties as the other two capture probes (9,9') and having a complementary sequence to another portion (4) of the target nucleotide sequence (8) except for one base (21) not being complementary, and

      - carrier means embedded computerized code for determining (detecting) the presence of the nucleotide base (1) at a given locus by a signal value of detection of the target sequence (7 or 7') on their specific capture probe (9 or 9'), which is higher or equal to a cut off signal value calculated from the signal value of detection of the target sequence (8) on their specific mutated capture probe (10) that gives a positive result identifying the nucleotide base (1).

25. The kit according to claim 24, wherein the insoluble solid support surface contains at least 10 different capture probes (10) targeting 5 different loci of gene(s).

26. The kit according to any of the claims 24 to 25, wherein the capture probes (9 or 9' and 10) differ by one base located at 4 to 10 bases from one extremity of the target specific part of the bound capture probe.

**27.** The kit according to any of the claims 24 to 26, wherein the capture probes (9, 9' and 10) are covalently attached to the surface of the solid support (22) by their 5' end.

**28.** The kit according to any of the claims 24 to 27, wherein the carrier means embedded computerized code determine that the organism is heterozygote at given locus when the signal values of detection of the target nucleotide sequences (7 and 7') upon mutated and non mutated capture probes (9 and 9') are both positive.

**29.** The kit according to claim 28, wherein the carrier means embedded computerized code determine that the organism is heterozygote at given locus when the signal values of detection of the target nucleotide sequences (7 and 7') upon mutated and non mutated capture probes (9 and 9') are both positive and furthermore that said signal values comply with the condition that the index calculated according to the formula:

$$CI = \frac{(N9 - N9')}{(N9 + N9')/2}$$

is lower than 1;
wherein N is the average signal value of detection upon mutated (9) or non mutated (9') capture probes.

**Patentansprüche**

**1.** Verfahren zum Bestimmen des Vorhandenseins oder Nichtvorhandenseins von mindestens 3 Einzelnukleotidpolymorphismen (Single Nucleotide Polymorphisms) bzw. SNP (s) (mutierte Base 1) an bestimmten Loci von (einer) Gennukleotidsequenz(en) (3) eines Organismus, welches folgende Schritte umfasst:

- Amplifizieren der homologen Gennukleotidsequenz (en) oder Anteilen davon, welche verschiedene Loci enthalten, zu einem ersten Satz/ersten Sätzen von Nukleotidzielsequenzen (homologe Sequenzen 7 bzw. 7') und;
- Amplifizieren eines zweiten Fragments der nicht mutierten Gennukleotidsequenz(en) zu einem zweiten Satz von Nukleotidzielsequenzen (8);
- Kontaktieren des ersten Satzes/der ersten Sätze der Nukleotidzielsequenzen (7 bzw. 7') mit einer Anordnung von Erfassungssonden (9, 9', 10), die an die Oberfläche eines festen Trägers (22) gebunden sind, wobei die Anordnung eine Reihe von Paaren von Erfassungssonden (9 bzw. 9') aufweist, welche dieselbe spezifische Hybridisierungssequenz aufweist, die zu einem Teil (2) der verschiedenen Nukleotidzielsequenzen (7 und 7') komplementär ist, sich aber durch eine einzelne Base (20), welche zu der in der zu charakterisierenden Locussequenz vorhandenen mutierten Base (1) komplementär ist, unterscheidet;
- Kontaktieren des zweiten Satzes der Nukleotidzielsequenz (8) mit einer komplementären Erfassungssonde (10), welche eine spezifische Hybridisierungssequenz aufweist, die zu einem Teil (4) der Nukleotidzielsequenz (8) komplementär ist, sich aber durch eine einzelne Base (21) unterscheidet;
- Feststellen und/oder Quantifizieren des Signalwertes der Hybridisierung des ersten Satzes der Nukleotidzielsequenzen (7 bzw. 7') und des Signalwertes der Hybridisierung des zweiten Satzes von Nukleotidzielsequenzen (8);
- Feststellen des Vorhandenseins der Nukleotidbase (1) an den unterschiedlichen Loci, wobei Signalwerte der Feststellung der Zielsequenz (en) (7 und/oder 7') auf den entsprechenden Erfassungssonden (9 bzw. 9') höher sind als bzw. gleich sind wie ein Grenzsignalwert, der aus dem Signalwert der Feststellung des zweiten Satzes der Zielsequenz (8) auf ihrer entsprechenden spezifischen mutierten Erfassungssonde (10) berechnet wird, wobei die verschiedenen Erfassungssonden (9, 9' und 10) ähnliche physikalische und chemische Eigenschaften aufweisen; und
- Feststellen des Vorhandenseins von Einzelnukleotidpolymorphismen (mutierte Base 1) an den verschiedenen Loci.

**2.** Verfahren nach Anspruch 1, wobei ähnliche physikalische und chemische Eigenschaften der Erfassungssonden (9, 9' und 10) einen GC-Gehalt zwischen etwa 40 und etwa 70% aufweisen.

**3.** Verfahren nach Anspruch 1 oder 2, wobei die Länge der Erfassungssonde (9, 9' und 10) zwischen etwa 15 und etwa 40 Basen umfasst.

**4.** Verfahren nach einem der vorherigen Ansprüche, wobei die Erfassungssonden eine Tm zwischen etwa 55°C und etwa 75°C aufweisen.

**5.** Verfahren nach einem der vorherigen Ansprüche, wobei die Hybridisierungstemperatur vorzugsweise zwischen 1°C und 10°C niedriger ist als die Schmelztemperatur (Tm) der spezifischen Sequenz der Erfassungssonden (9, 9' und 10) .

**6.** Verfahren nach einem der vorherigen Ansprüche, wobei die Erfassungssonden über ihr 5'-Ende kovalent an der Oberfläche des festen Trägers befestigt sind.

**7.** Verfahren nach einem der vorherigen Ansprüche, wobei sich die Erfassungssonden (9 oder 9' oder 10) durch eine Base unterscheiden, die sich in einem Abstand von etwa 4 bis etwa 10 Basen vom 3'-Ende des zielspezifischen Teils der gebundenen Erfassungssonde entfernt befindet.

**8.** Verfahren nach einem der vorherigen Ansprüche, wobei es sich bei dem Grenzsignalwert um den Signalwert der Feststellung des zweiten Satzes von Nukleotidzielsequenzen (8) auf ihrer entsprechenden spezifischen mutierten Erfassungssonde (10), multipliziert mit einem Faktor zwischen 2 und 5, handelt.

**9.** Verfahren nach Anspruch 8, wobei der Grenzsignalwert aus einem durchschnittlichen Signalwert der Feststellung von mindestens zwei verschiedenen Sätzen von Nukleotidzielsequenzen (8) auf mindestens zwei verschiedenen komplementären spezifischen mutierten Erfassungssonden (10) berechnet wird.

**10.** Verfahren nach Anspruch 8 oder 9, wobei der Grenzsignalwert aus einem durchschnittlichen Signalwert der Feststellung von mindestens zwei verschiedenen Sätzen von Nukleotidzielsequenzen (7 bzw. 7') auf mindestens zwei verschiedenen komplementären spezifischen mutierten Erfassungssonden (9 bzw. 9') berechnet wird.

**11.** Verfahren nach einem der vorherigen Ansprüche, wobei der Organismus als an bestimmten Loci heterozygot betrachtet wird, wenn die Signalwerte der Feststellung der Nukleotidzielsequenzen (7 und 7') sowohl auf mutierten als auch auf nicht-mutierten Erfassungssonden (9 und 9') positiv sind.

**12.** Verfahren nach Anspruch 11, wobei der Organismus als an bestimmten Loci heterozygot betrachtet wird, wenn die Signalwerte der Feststellung der Nukleotidzielsequenzen (7 und 7') sowohl auf mutierten als auch auf nicht mutierten Erfassungssonden (9 und 9') positiv sind und darüber hinaus diese Signalwerte die Bedingung erfüllen, dass der nach der Formel

$$CI \quad \frac{(N9-N9')}{(N9+N9')/2}$$

berechnete Index kleiner ist als 1;
wobei es sich bei N um den durchschnittlichen Signalwert der Feststellung auf mutierten (9) bzw. nicht-mutierten (9') Erfassungssonden handelt.

**13.** Verfahren nach Anspruch 12, wobei der CT-Wert kleiner ist als 0,7.

**14.** Verfahren nach einem der vorherigen Ansprüche, wobei der erste und der zweite Satz von Nukleotidzielsequenzen (7 bzw. 7' und 8) durch Amplifizierung mit demselben Primerpaar (5, 6') erhalten wird.

**15.** Verfahren nach einem der Ansprüche 1 bis 13, wobei der erste und der zweite Satz von Nukleotidzielsequenzen (7 bzw. 7' und 8), erhalten durch Amplifizierung mit einem anderen Primerpaar (5, 5' und 6, 6'), ein Feststellungssignal auf ihren spezifischen Erfassungssonden ergeben, das identisch ist oder sich um einen Faktor von weniger als 3 unterscheidet.

**16.** Verfahren nach einem der vorherigen Ansprüche zum Feststellen von SNP in den menschlichen Cytochromen P450 2C9, 2C19 und 2D6.

**17.** Verfahren nach Anspruch 16, wobei die Cytochrome p450, 2C9 und 2D6 auf einer Anordnung von Erfassungssonden (9, 9', 10) festgestellt werden, die spezifische Sequenzen enthalten, wie sie in Tabelle 1 bereitgestellt sind.

**18.** Verfahren nach einem der vorherigen Ansprüche, wobei die Nukleotidzielsequenzen während der Amplifizierung markiert werden.

**19.** Verfahren nach einem der vorherigen Ansprüche, wobei die Nukleotidzielsequenzen nach der Amplifizierung fragmentiert werden.

**20.** Verfahren nach Anspruch 19, wobei die Nukleotidzielsequenzen durch DNase-Behandlung fragmentiert werden.

**21.** Verfahren nach einem der vorherigen Ansprüche, wobei die Erfassungssonden eine spezifische Sequenz für die Bindung an die Nukleotidzielsequenz aufweisen, welche durch einen Abstandshalter mit dem festen Träger (22) verknüpft ist.

**22.** Verfahren nach Anspruch 21, wobei der Abstandshalter ein Polynukleotid mit einer Länge von mindestens etwa 20 Nukleotiden ist.

**23.** Verfahren nach Anspruch 21 oder 22, wobei der Abstandshalter ein Polynukleotid mit einer Länge von mindestens etwa 90 Nukleotiden ist.

**24.** Kit zum Feststellen einer Nukleotidbase (1) an einem bestimmten Locus einer Gennukleotidsequenz (3) eines Organismus, umfassend:

- eine Fläche eines unlöslichen festen Trägers (22), vorzugsweise eine nicht-poröse, feste Trägerfläche, umfassend:

(a) mindestens zwei Erfassungssonden (9, 9'), gebunden an bestimmten Stellen der Fläche eines unlöslichen festen Trägers (22), wobei die Erfassungssonden (9, 9') eine spezifische Sequenzkomplementarität zu einem Teil (2) einer Nukleotidzielsequenz (7 bzw. 7') aufweisen, die einen ersten Locus aufweist, und wobei sich jede Erfassungssonde (9) von der anderen Erfassungssonde (9') um eine einzige Base (20) an der Locusstelle unterscheidet;

(b) mindestens eine weitere Erfassungssonde (10), die sich extern zu dem ersten Locus befindet und ähnliche physikalische und chemische Eigenschaften wie die anderen beiden Erfassungssonden (9, 9') und eine zu einem anderen Teil (4) der Nukleotidzielsequenz (8) komplementäre Sequenz aufweist, ausgenommen eine Base (21), die nicht komplementär ist, und

- einen in das Trägermittel eingebetteten computerisierten Code zum Feststellen (Erfassen) des Vorhandenseins der Nukleotidbase (1) an einem bestimmten Locus durch einen Signalwert der Feststellung der Zielsequenz (7 bzw. 7') auf ihrer spezifischen Erfassungssonde (9 bzw. 9'), welcher höher ist als oder gleich ist wie ein Grenzsignalwert, der aus dem Signalwert der Feststellung der Zielsequenz (8) auf ihrer spezifischen mutierten Erfassungssonde (10) errechnet wird, der ein positives Ergebnis ergibt, wodurch die Nukleotidbase (1) identifiziert wird.

**25.** Kit nach Anspruch 24, wobei die unlösliche feste Trägerfläche mindestens 10 verschiedene Erfassungssonden (10) enthält, welche gegen 5 verschiedene Genloci gerichtet sind.

**26.** Kit nach einem der Ansprüche 24 bis 25, wobei sich die Erfassungssonden (9 bzw. 9' und 10) durch eine Base unterscheiden, die sich 4 bis 10 Basen von einer Extremität des zielspezifischen Teils der gebundenen Erfassungssonde entfernt befindet.

**27.** Kit nach einem der Ansprüche 24 bis 26, wobei die Erfassungssonden (9 bzw. 9' und 10) über ihr 5'-Ende kovalent an der Oberfläche des festen Trägers (22) befestigt sind.

**28.** Kit nach einem der Ansprüche 24 bis 27, wobei der in das Trägermittel eingebettete computerisierten Code bestimmt, dass der Organismus an einem bestimmten Locus heterozygot ist, wenn die Signalwerte der Feststellung der Nukleotidzielsequenzen (7 und 7') auf mutierten und nicht-mutierten Erfassungssonden (9 und 9') beide positiv sind.

**29.** Kit nach Anspruch 28, wobei der in das Trägermittel eingebettete computerisierte Code bestimmt, dass der Organismus an einem bestimmten Locus heterozygot ist, wenn die Signalwerte der Feststellung der Nukleotidzielsequenzen (7 und 7') auf mutierten und nicht-mutierten Erfassungssonden (9 und 9') beide positiv sind und darüber hinaus die Signalwerte die Bedingung erfüllen, dass der nach der Formel

$$CI \quad \frac{(N9-N9')}{(N9+N9')/2}$$

berechnete Index kleiner ist als 1;
wobei es sich bei N um den durchschnittlichen Signalwert der Feststellung auf mutierten (9) bzw. nicht-mutierten (9') Erfassungssonden handelt.

**Revendications**

1. Procédé pour déterminer la présence ou l'absence d'au moins 3 polymorphismes d'un seul nucléotide ou SNP (1 base mutée) à des loci donnés d'une/de séquence(s) nucléotidique (s) d'un gène (3) d'un organisme et comprenant les étapes consistant à :

   - amplifier la/les séquence(s) nucléotidique(s) d'un gène homologue (s) ou une partie de celle (s)-ci contenant différents loci dans un/des premier(s) ensemble (s) de séquences nucléotidiques cibles (séquences homologues 7 ou 7'), et ;
   - amplifier un second fragment de la/des séquence(s) nucléotidique (s) d'un gène qui est/sont non mutée (s) dans un second ensemble de séquences nucléotidiques cibles (8) ;
   - mettre en contact le/les premier (s) ensemble(s) de séquences nucléotidiques cibles (7 ou 7') sur un réseau de sonde de captures (9, 9', 10) liées à la surface d'un support solide (22), ledit réseau comprenant une série de paires de sondes de capture (9 et 9') ayant la même séquence d'hybridation spécifique complémentaire à une partie (2) des différentes séquences nucléotidiques cibles (7 et 7'), mais étant différentes par une seule base (20) qui est complémentaire à la base mutée (1), présente dans la séquence de locus devant être

   **caractérisée** ;

   - mettre en contact le second ensemble de séquences nucléotidiques cibles (8) avec une sonde de capture complémentaire (10) ayant une séquence d'hybridation spécifique complémentaire à une partie (4) de ladite séquence nucléotidique cible (8), mais étant différente par une seule base (21) ;
   - détecter et/ou quantifier la valeur de signal de l'hybridation du premier ensemble de séquences nucléotidiques cibles (7 ou 7') et la valeur de signal de l'hybridation du second ensemble de séquences nucléotidiques cibles (8) ;
   - déterminer la présence de la base nucléotidique (1) auxdits différents loci, où des valeurs de signal de détection de la/des séquence(s) cible(s) (7 et/ou 7') sur leurs sondes de capture correspondantes (9 ou 9') sont supérieures ou égales à une valeur de signal seuil calculée à partir de la valeur de signal de détection du second ensemble de la séquence cible (8) sur sa sonde de capture mutée spécifique correspondante (10) ; où les différentes sondes de capture (9, 9' et 10) possèdent des propriétés physiques et chimiques similaires, et ;
   - déterminer la présence de polymorphismes d'un seul nucléotide (1 base mutée) dans lesdits différents loci.

2. Procédé selon la revendication 1, où des propriétés physiques et chimiques similaires des sondes de capture (9, 9' et 10) présentent un contenu en GC compris entre environ 40 et environ 70 %.

3. Procédé selon la revendication 1 ou 2, où la longueur de la sonde de capture (9, 9' et 10) est comprise entre environ is et environ 40 bases.

4. Procédé selon l'une quelconque des revendications précédentes, où les sondes de capture possèdent une Tm comprise entre environ 55 et environ 75 °C.

5. Procédé selon l'une quelconque des revendications précédentes, où la température d'hybridation est de préférence comprise entre 1 et 10°C, en étant inférieure à la température de fusion (Tm) de la séquence spécifique des sondes de capture (9, 9' et 10).

6. Procédé selon l'une quelconque des revendications précédentes, où les sondes de capture sont attachées de manière covalente à la surface du support solide par leur extrémité 5'.

**7.** Procédé selon l'une quelconque des revendications précédentes, où les sondes de capture (9 ou 9' et 10) diffèrent par une base qui est localisée à une distance d'environ 4 à environ 10 bases par rapport à l'extrémité 3' de la partie spécifique cible de la sonde de capture liée.

**8.** Procédé selon l'une quelconque des revendications précédentes, où la valeur de signal seuil est la valeur de signal de détection du second ensemble de séquences nucléotidiques cibles (8) sur sa sonde de capture mutée spécifique correspondante (10) multipliée par un facteur compris entre 2 et 5.

**9.** Procédé selon la revendication 8, où la valeur de signal seuil est calculée à partir d'une valeur de signal de détection moyenne d'au moins deux ensembles différents de séquences nucléotidiques cibles (8) sur au moins deux différentes sondes de capture mutées spécifiques complémentaires (10).

**10.** Procédé selon la revendication 8 ou 9, où la valeur de signal seuil est calculée à partir d'une valeur de signal de détection moyenne d'au moins deux ensembles différents de séquences nucléotidiques (7 ou 7') sur au moins deux différentes sondes de capture mutées spécifiques complémentaires (9 ou 9').

**11.** Procédé selon l'une quelconque des revendications précédentes, où l'organisme est considéré comme hétérozygote à des loci donnés lorsque les valeurs de signal de détection des séquences nucléotidiques cibles (7 et 7') sur des sondes de capture mutées et non mutées (9 et 9') sont toutes deux positives.

**12.** Procédé selon la revendication 11, où l'organisme est considéré comme hétérozygote à des loci donnés lorsque les valeurs de signal de détection des séquences nucléotidiques cibles (7 et 7') sur des sondes de capture mutées et non mutées (9 et 9') sont toutes deux positives et en outre lorsque lesdites valeurs de signal sont conformes à la condition qui est que l'indice calculé selon la formule :

$$CI = \frac{(N9 - N9')}{(N9 + N9')/2} \, ,$$

est inférieur à 1 ;
où N est la valeur de signal de détection moyenne sur des sondes de capture mutées (9) ou non mutées (9').

**13.** Procédé selon la revendication 12, où le CI est inférieur à 0,7.

**14.** Procédé selon l'une quelconque des revendications précédentes, où le premier et le second ensembles de séquences nucléotidiques cibles (7 ou 7' et 8) sont obtenus par une amplification avec la même paire d'amorces (5, 6').

**15.** Procédé selon l'une quelconque des revendications 1 à 13, où le premier et le second ensembles de séquences nucléotidiques cibles (7 ou 7' et 8) obtenus par une amplification avec une paire d'amorce différente (5, 5' et 6, 6') génèrent des signaux de détection sur leurs sondes de capture spécifiques qui sont identiques ou diffèrent par un facteur inférieur à 3.

**16.** Procédé selon l'une quelconque des revendications précédentes, pour la détection de SNP dans les cytochromes humains P450, 2C9, 2C19 et 2D6.

**17.** Procédé selon la revendication 16, où les cytochromes P450, 2C9 et 2D6 sont détectés sur un réseau de sondes de capture (9, 9', 10) contenant des séquences spécifiques tel que montré dans le tableau 1.

**18.** Procédé selon l'une quelconque des revendications précédentes, où les séquences nucléotidiques cibles sont marquées pendant l'amplification.

**19.** Procédé selon l'une quelconque des revendications précédentes, où les séquences nucléotidiques cibles sont fragmentées après l'amplification.

**20.** Procédé selon la revendication 19, où les séquences nucléotidiques cibles sont fragmentées par un traitement par la DNase.

**21.** Procédé selon l'une quelconque des revendications précédentes, où les sondes de capture comprennent une séquence spécifique pour s'attacher à la séquence nucléotidique cible liée à la surface du support solide (22) au moyen un espaceur.

22. Procédé selon la revendication 21, où l'espaceur est un polynucléotide ayant une longueur d'au moins environ 20 nucléotides.

23. Procédé selon la revendication 21 ou 22, où l'espaceur est un polynucléotide ayant une longueur d'au moins environ 90 nucléotides.

24. Kit pour la détection d'une base nucléotidique (1) à un locus donné d'une séquence nucléotidique d'un gène (3) d'un organisme comprenant :

   - une surface de support solide insoluble (22), de préférence une surface de support solide non poreux comprenant :

   (a) au moins deux sondes de capture (9, 9') liées à des emplacements particuliers de ladite surface de support solide insoluble (22), lesdites sondes de capture (9, 9') ayant une séquence spécifique complémentaire à une partie (2) d'une séquence nucléotidique cible (7 ou 7') comprenant un premier locus et où chaque sonde de capture (9) diffère de l'autre sonde de capture (9') par une seule base (20) au niveau du site du locus ;
   (b) au moins une sonde de capture supplémentaire (10) qui est située à l'extérieur du premier locus et possède des propriétés physiques et chimiques similaires aux deux autres sondes de capture (9, 9'), et qui possède une séquence complémentaire à une autre partie (4) de la séquence nucléotidique cible (8) à l'exception d'une base (2') qui n'est pas complémentaire, et,

   - un code informatisé incorporé dans un moyen de transport pour déterminer (détecter) la présence de la base nucléotidique (1) à un locus donné par une valeur de signal de détection de la séquence cible (7 ou 7') sur sa sonde de capture spécifique (9 ou 9'), qui est supérieure ou égale à une valeur de signal seuil calculée à partir de la valeur de signal de détection de la séquence cible (8) sur sa sonde de capture mutée spécifique (10) qui génère un résultat positif identifiant la base nucléotidique (1).

25. Kit selon la revendication 24, où la surface du support solide insoluble contient au moins 10 sondes de capture différentes (10) ciblant 5 loci différents de gène(s).

26. Kit selon l'une quelconque des revendications 24 à 25, où les sondes de capture (9 ou 9' et 10) diffèrent par une base située à 4 à 10 bases d'une extrémité de la partie spécifique cible de la sonde de capture liée.

27. Kit selon l'une quelconque des revendications 24 à 26, où les sondes de capture (9, 9' et 10) sont attachées de manière covalente à la surface du support solide (22) par leur extrémité 5'.

28. Kit selon l'une quelconque des revendications 24 à 27, où le code informatisé incorporé dans un moyen de transport détermine que l'organisme est hétérozygote à un locus donné lorsque les valeurs de signal de détection des séquences nucléotidiques cibles (7 et 7') sur des sondes de capture mutées et non mutées (9 et 9') sont toutes deux positives.

29. Kit selon la revendication 28, où le code informatisé incorporé dans un moyen de transport détermine que l'organisme est hétérozygote à un locus donné lorsque les valeurs de signal de détection des séquences nucléotidiques cibles (7 et 7') sur des sondes de capture mutées et non mutées (9 et 9') sont toutes deux positives et en outre lorsque lesdites valeurs de signal sont conformes à la condition qui est que l'indice calculé selon la formule :

$$CI = \frac{(N9 - N9')}{(N9 + N9')/2},$$

est inférieur à 1 ;
où N est la valeur de signal de détection moyenne sur des sondes de capture mutées (9) ou non mutées (9').

FIG.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6410229 B
- WO 9729212 A
- US 5858659 A
- US 20050089877 A
- US 6852488 B
- WO 9727317 A
- EP 1096024 A
- WO 9932660 A
- US 20050095635 A
- WO 9710365 A
- WO 9828444 A
- US 5312527 A
- EP 0535242 A
- US 5736257 A
- WO 9935499 A
- US 5552270 A
- WO 0218288 A
- WO 0177372 A

**Non-patent literature cited in the description**

- **WEN SY et al.** *World J. Gastroenterol.,* 2003, vol. 9, 1342-1346
- **GRIMM et al.** *Journal of Clinical Microbioloy,* 2004, vol. 42, 3766-3774

SEQUENCE LISTING

<110> Eppendorf Array Technologies SA

<120> Detection method of homologous sequences differing by one base on a microarray

<130> BP.EATE.009A/EP

<160> 30

<170> PatentIn version 3.3

<210> 1
<211> 24
<212> DNA
<213> Artificial Sequence

<220>
<223> MP2C19 06 primer

<400> 1
agcaatggaa agaratggaa ggag          24

<210> 2
<211> 23
<212> DNA
<213> Artificial Sequence

<220>
<223> MP2C9 07 primer

<400> 2
gaaaccccag agaagtcagt gag          23

<210> 3
<211> 22
<212> DNA
<213> Artificial sequence

<220>
<223> MP2D6 01 primer

<400> 3
cgcttctccg tgtccacctt gc          22

<210> 4
<211> 23
<212> DNA
<213> Artificial sequence

<220>
<223> MP2D6 02 primer

<400> 4
gccactctca ccttctccat ctc          23

<210> 5
<211> 25
<212> DNA
<213> Artificial sequence

<220>
<223> MP2C9 06 primer

<400> 5
gctttgtact atcaatcagg ttgtc          25

<210> 6
<211> 26
<212> DNA
<213> Artificial Sequence

<220>
<223> MP2C9 02 primer

<400> 6
cacaaattca caagcagtca cataac          26

<210> 7
<211> 25
<212> DNA
<213> Artificial Sequence

<220>
<223> MP2C9 03 primer

<400> 7
ctaaagtcca ggaagagatt gaacg          25

<210> 8
<211> 27
<212> DNA
<213> Artificial Sequence

<220>
<223> MP2C9 04 primer

<400> 8
cagagtgttg atttgacaag attttac          27

<210> 9
<211> 21
<212> DNA
<213> Artificial Sequence

<220>
<223> 2D6*1,3 probe

<400> 9
ctgctaactg agcacaggat g          21

<210> 10
<211> 21
<212> DNA
<213> Artificial Sequence

<220>
<223> 2D6*3 probe

<400> 10
ctgctaactg agcacggatg a          21

<210> 11
<211> 19
<212> DNA

<213> Artificial Sequence

<220>
<223> 2D6*1,4 probe

<400> 11
gcgaaagggg cgtcctggg          19

<210> 12
<211> 19
<212> DNA
<213> Artificial sequence

<220>
<223> 2D6*4 probe

<400> 12
gcgaaagggg cgtcttggg          19

<210> 13
<211> 19
<212> DNA
<213> Artificial sequence

<220>
<223> 2D6*1,6 probe

<400> 13
cctcggtcac ccactgctc          19

<210> 14
<211> 19
<212> DNA
<213> Artificial sequence

<220>
<223> 2D6*6 probe

<400> 14
tcctcggtca cccctgctc          19

<210> 15
<211> 20
<212> DNA
<213> Artificial Sequence

<220>
<223> 2D6 control probe

<400> 15
gttggcgaag gcggcacaaa          20

<210> 16
<211> 21
<212> DNA
<213> Artificial Sequence

<220>

<223> 2D6 external probe

<400> 16
gttggcgaag gcggaacaaa g        21

<210> 17
<211> 22
<212> DNA
<213> Artificial sequence

<220>
<223> 2c9*1,9 probe

<400> 17
tgtccattga ttcttggtgt tc        22

<210> 18
<211> 21
<212> DNA
<213> Artificial Sequence

<220>
<223> 2C9*9 probe

<400> 18
gtccattgat tcttggcgtt c        21

<210> 19
<211> 23
<212> DNA
<213> Artificial Sequence

<220>
<223> 2C9*1,10 probe

<400> 19
cttcctgatg aaaatggaga agg        23

<210> 20
<211> 23
<212> DNA
<213> Artificial Sequence

<220>
<223> 2C9*10 probe

<400> 20
cttcctgatg aaaatgggga agg        23

<210> 21
<211> 21
<212> DNA
<213> Artificial sequence

<220>
<223> 2C9*1,3=*1,4 probe

<400> 21

ggtggggaga aggtcaatgt a          21

<210> 22
<211> 21
<212> DNA
<213> Artificial sequence

<220>
<223> 2C9*3 probe

<400> 22
ggtggggaga aggtcaaggt a          21

<210> 23
<211> 21
<212> DNA
<213> Artificial Sequence

<220>
<223> 2C9*1,4 probe

<400> 23
ggtggggaga aggtcaatgt a          21

<210> 24
<211> 21
<212> DNA
<213> Artificial sequence

<220>
<223> 2C9*4 probe

<400> 24
ggtggggaga aggtcagtgt a          21

<210> 25
<211> 20
<212> DNA
<213> Artificial Sequence

<220>
<223> 2C9*1,5 probe

<400> 25
gctggtgggg agaaggtcaa            20

<210> 26
<211> 20
<212> DNA
<213> Artificial Sequence

<220>
<223> 2C9*5 probe

<400> 26
gctggtgggg agaagctcaa            20

<210> 27

<211> 20
<212> DNA
<213> Artificial Sequence

<220>
<223> 2C9*1,11 probe

<400> 27
atgcaggggc tccggtttct      20

<210> 28
<211> 20
<212> DNA
<213> Artificial Sequence

<220>
<223> 2C9*11 probe

<400> 28
catgcagggg ctccagtttc      **2**0

<210> 29
<211> 19
<212> DNA
<213> Artificial sequence

<220>
<223> 2C9 control probe

<400> 29
gcaggctggt ggggagaag      19

<210> 30
<211> 19
<212> DNA
<213> Artificial sequence

<220>
<223> 2C9 external probe

<400> 30
gcaggctggt gggaagaag      19